# DEMANDE DE BREVET EUROPEEN

(11) **EP 4 420 687 A1**
(43) Date de publication de la demande: **28.08.2024**
(21) Numéro de dépôt: 24305288.3
(22) Date de dépôt: 22.02.2024
(51) Int. Cl.: A61L 2/10, C02F 1/32

(54) **DISPOSITIF DE TRAITEMENT D'UN LIQUIDE PAR RAYONNEMENT UV-C ET FONTAINE À EAU LE COMPORTANT**

(30) Priorité: 24.02.2023 FR 2301723
(71) Demandeur: Mistral Constructeur, 91130 Ris Orangis (FR)
(72) Inventeur: LICCIONI, Thomas, 92330 SCEAUX (FR)
(74) Mandataire: Santarelli

(57) **Abrégé**

Dispositif de traitement d'un liquide par rayonnement UV-C et fontaine à eau le comportant.

Le dispositif comporte une chambre de traitement du liquide (6), au moins une LED (22) émettant de la lumière UV-C , pour irradier le liquide à traiter circulant dans la chambre de traitement (6) avec son rayonnement UV-C une pluralité de canaux d'entrée (11) par lesquels le liquide à traiter pénètre dans la chambre de traitement (6) et un orifice de sortie (12) de la chambre de traitement (6), par lequel le liquide sort de la chambre de traitement (6) une fois traité, lesdits canaux d'entrée (11) étant disposés concentriquement autour de l'orifice de sortie de liquide (12) et suivant une configuration en tronc de cône dont la petite base fait face à l'au moins une LED (22).

## Description

La présente demande concerne les dispositifs de traitement d'un liquide par rayonnement ultraviolet (UV), comme par exemple l'eau.

De tels dispositifs sont notamment destinés à être disposés dans un appareil de distribution de boisson, comme par exemple une fontaine à eau, et en particulier près d'un point de distribution de la boisson.

Des dispositifs de traitement d'eau par rayonnement UV sont par exemple connus des demandes de brevets DE202022104664, US2019/0225509 et US11,104,590 Il reste nécessaire d'augmenter le niveau d'irradiation avec la lumière ultraviolette irradiant le liquide au moyen de tels dispositifs, afin d'améliorer l'effet de désinfection de l'irradiation. Cependant, l'augmentation du nombre de dispositifs émetteurs de lumière pour obtenir un effet de désinfection approprié entraîne une augmentation du coût.

La présente invention vise à améliorer les dispositifs existants et mener en outre à d'autres avantages.

Elle propose à cet effet un dispositif de traitement d'un liquide par rayonnement UV-C, comportant une chambre de traitement du liquide, au moins une LED (c'est-à-dire une diode électroluminescente) émettant de la lumière UV-C, pour irradier le liquide à traiter circulant dans la chambre avec son rayonnement UV- C, une pluralité de canaux d'entrée par lesquels le liquide à traiter pénètre dans la chambre de traitement et une sortie par lequel le liquide sort de la chambre de traitement une fois traité, lesdits canaux d'entrée étant disposés concentriquement autour de la sortie de liquide et suivant une configuration en tronc de cône dont la petite base fait face à l'au moins une LED.

Grâce à ces dispositions, le flux d'eau pénétrant dans la chambre de traitement est dirigé sous forme de jets sur la source de lumière UV-C (la ou les LEDs) et plus précisément vers le sommet du cône de lumière formé par la ou les LEDs, où l'intensité lumineuse est maximale, à la faveur d'une désinfection avec une efficacité maximale.

Suivant une disposition originale en soi, car pouvant être mise en oeuvre sans la configuration spécifique des canaux d'entrée et de la sortie de liquide, telle que définie supra, le dispositif comporte un canal de refroidissement entourant un échangeur thermique sur lequel est fixé une carte électronique portant la ou les LEDs, le canal de refroidissement communiquant fluidiquement avec une arrivée de liquide à traiter dans le dispositif de traitement à l'une de ses extrémités et avec la pluralité de canaux d'entrée à son autre extrémité.

Le dispositif selon l'invention peut comprendre en outre tout ou partie des caractéristiques suivantes, pouvant être combinées :
- le dispositif comporte un passage d'écoulement de liquide traité s'étendant de l'orifice de sortie de la chambre de traitement jusqu'à un point de distribution du liquide traité ;
- la chambre et, le cas échéant, le passage d'écoulement, présentent une surface interne réfléchissant le rayonnement UV-C jusqu'au point de distribution ;
- la chambre et, le cas échéant, le passage d'écoulement, sont formés d'un matériau réfléchissant le rayonnement UV-C, de préférence un fluoropolymère, tel que du polytétrafluoroéthylène (PTFE) ;
- la au moins une LED est située dans un logement étanche aux fluides, séparé de la chambre de traitement par une fenêtre transparente au rayonnement UV-C ;
- le canal de refroidissement est formé entre un corps dans lequel sont logés la chambre et l'échangeur thermique, et ces derniers ;
- le corps présente intérieurement des nervures espaçant le corps de la chambre de traitement ;
- le passage d'écoulement comporte un premier tube et les canaux d'entrée sont formés par des gorges ménagées dans le bord de l'orifice de sortie et une extrémité du premier tube logée dans l'orifice ;
- le passage d'écoulement comporte un premier tube puis un second tube formant un angle avec le premier tube, et un point de distribution du liquide traité ;
- le passage d'écoulement est partiellement tronconique dans le premier tube et, le cas échéant, le second tube ;
- l'axe de la partie du passage d'écoulement située dans le premier tube forme un angle supérieur à 90°, de préférence égal à 100°, avec l'axe de la partie du passage d'écoulement située dans le second tube et une surface oblique faisant face à la partie de passage d'écoulement située dans le premier tube opère la jonction entre les deux parties de passage d'écoulement ;
- le premier tube ou les premier et second tubes sont logés dans une tête de distribution montée amovible sur le corps avec possibilité de montage selon différentes positions angulaires d'un tube d'arrivée de liquide dans le dispositif, raccordé au corps .

Selon un autre aspect de la présente invention, est également proposée une fontaine à eau comportant un dispositif tel que décrit précédemment, avec tout ou partie des caractéristiques précitées et apportant des avantages analogues.

Suivant des dispositions spécifiques de cette fontaine, pouvant être combinées :
- le dispositif de traitement est monté sur la fontaine de sorte que la chambre de traitement est agencée inclinée vers le bas ;
- la fontaine comporte des moyens de commande pour un allumage cyclique de la au moins une LED, destiné empêcher une rétro-contamination entre le point de distribution et le reste du dispositif de traitement.

D'autres avantages et caractéristiques de la présente invention ressortiront à la lecture détaillée qui suit, en référence aux dessins annexés, donnés à titre illustratif et nullement limitatif, parmi lesquels :
la [Fig. 1] représente un dispositif de traitement selon un mode de réalisation de la présente invention ;
la [Fig. 2] présente le dispositif de la figure 1 en éclaté ;
la [Fig. 3] est une vue en coupe du dispositif des figures 1 et 2 ;
la [Fig. 4] représente le dispositif de la figure 3 avec une illustration de la circulation du liquide et du cheminement des rayons UV ;
la [Fig. 5] est une vue schématique en perspective illustrant les différentes positions possibles du tube d'entrée d'eau dans le dispositif de traitement ;et
La [Fig. 6] est une vue schématique partielle d'une fontaine à eau comportant un dispositif selon un mode de réalisation de la présente invention, positionné de manière inclinée.

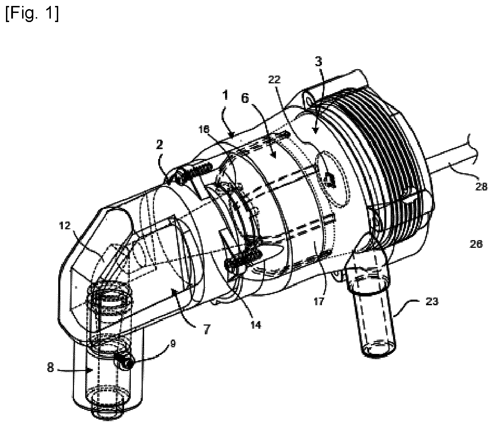

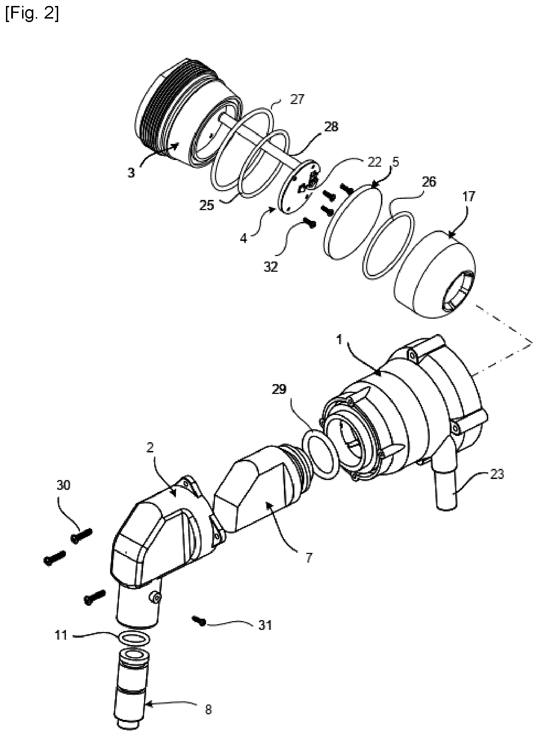

Comme l'illustrent principalement les figures 1 à 5, un dispositif de traitement selon un mode de réalisation de la présente invention est un système de désinfection d'un liquide, tel que de l'eau, s'écoulant à travers lui.

Selon ce mode de réalisation, le dispositif de traitement du liquide par rayonnement ultraviolet comporte un réacteur de désinfection ayant un corps 1 de réacteur, sur lequel est montée une tête orientable 2 de réacteur.

D'une extrémité d'entrée de liquide à traiter dans le dispositif de traitement à un point de distribution du liquide sont successivement logés dans le corps de réacteur, un échangeur thermique 3 pour refroidir une carte électronique 4, puis cette carte électronique sur laquelle est montée une LED 22 émettant de la lumière UV-C pour irradier le liquide à traiter circulant dans le dispositif de traitement et doté d'un circuit d'alimentation de celle-ci, d'une fenêtre 5 transparente aux UV-C , d'une chambre de traitement 6 réfléchissant la lumière UV-C, puis dans la tête orientable 2, une tête intérieure 7 ou tube de raccordement fluidique de la chambre de traitement 6 à un tube 8 de sortie de liquide formant point de distribution du liquide traité.

Ce tube 8 de sortie de liquide est emboîté dans la tête intérieure 7, et fixé par une vis 9 à la tête orientable 2, un joint d'étanchéité 10 assurant l'étanchéité à la jonction du tube 8 de sortie et de la tête intérieure 7.

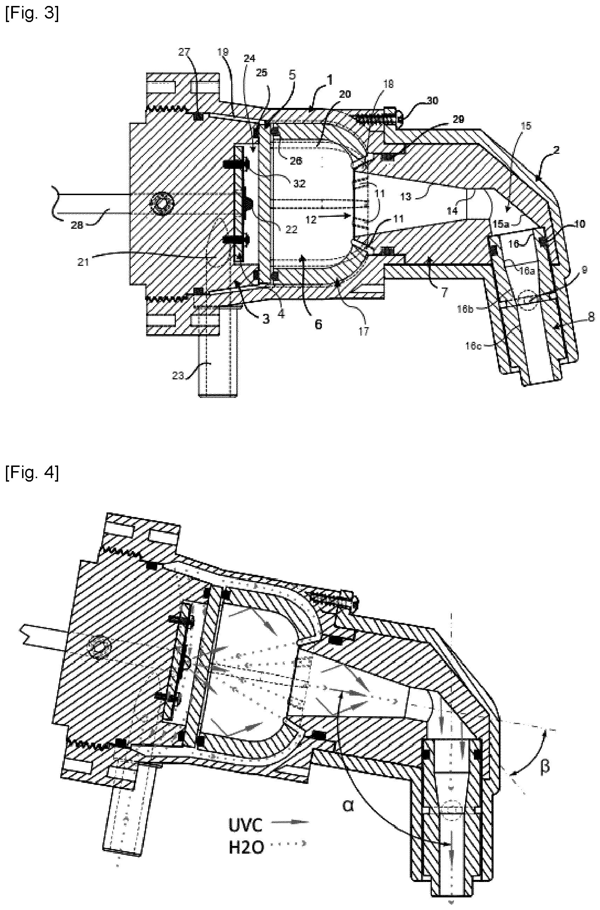

A l'autre extrémité, le liquide à traiter pénètre dans la chambre de traitement 6 par une pluralité de canaux d'entrée 11 et en ressort par un orifice de sortie 12 une fois traité. Comme illustré plus précisément sur les figures 3 et 4, ces canaux d'entrée 11 sont disposés concentriquement autour de l'orifice de sortie 12 et suivant une configuration en tronc de cône, dont la petite base faite face à la LED 22 émettant de la lumière UV-C.

Ainsi, comme mentionné supra, le flux d'eau pénétrant dans la chambre de traitement 6 est dirigé sous forme de jets sur la LED 22 et plus précisément vers le sommet du cône de lumière formée par celle-ci, c'est-à-dire en pratique son centre, où l'intensité lumineuse et maximale.

Le flux d'eau sort ensuite de la chambre de traitement 6 par l'orifice de sortie 12 de celle-ci et pénètre dans une seconde chambre de traitement formé par la tête intérieure 7 et ayant un passage tronconique 13 prolongé par un rétrécissement cylindrique 14 de cette tête, qui débouche sur un passage 15 de cette tête ayant une configuration générale en forme de prisme triangulaire, et qui précède un passage 16 du tube de sortie 8, lui-même formé par un tronçon cylindrique 16a suivi d'un tronçon tronconique 16b puis à nouveau d'un tronçon cylindrique 16c à l'extrémité du tube de sortie 8 formant le point de distribution.

On observera encore que la chambre de traitement 6 est formée par une coupelle 17 dont le fond est traversé par l'orifice de sortie 12, et dont le bord de l'orifice est muni de gorges régulièrement réparties sur sa circonférence et présente une section tronconique afin de former au moyen de ces gorges lesdits canaux d'entrée 11 disposés suivant une configuration en tronc de cône.

La tête intérieure 7 présente du côté de cet orifice 12 une couronne 18 de section triangulaire, venue de matière avec le reste de la tête intérieure 7 et logée dans l'orifice 12 de sorte à délimiter avec les gorges les canaux d'entrée 11.

Cette couronne 18 n'occupe pas toute l'épaisseur de la tête intérieure 7 et présente une face opposée à celle de la coupelle 17 dans laquelle sont ménagées les gorges ,qui a une largeur supérieure à celle de cette face de coupelle 17, afin de permettre aux canaux d'entrée 11 de communiquer fluidiquement avec un canal de refroidissement 19.

Plus précisément, le corps 1 de réacteur est pourvu intérieurement d'une pluralité de nervures 20 réparties sur sa circonférence, de sorte à laisser subsister entre la surface intérieure de ce corps 1 et la chambre de traitement 6, le canal de refroidissement 19, tout en centrant la coupelle 17 dans le corps 1.Ce dernier est raccordé fluidiquement à une entrée de liquide 21 ménagée dans le dispositif de traitement, à son extrémité opposée à celle communiquant avec la pluralité de canaux d'entrée 11 de liquide dans la chambre de traitement 6.

En pratique, un tube 23 d'entrée de liquide communique, dans le cadre du présent mode de réalisation, sur le côté du corps 1 de réacteur avec l'entrée 21 pour créer un tourbillon de liquide dans le canal de refroidissement 19 autour de l'échangeur thermique 3 servant à refroidir la carte électronique 4. Cet échangeur thermique 3 réalisé en un matériau très bon conducteur de chaleur, ici de l'aluminium revêtu ou de l'acier inoxydable, est ainsi constamment refroidi par le liquide circulant autour de lui.

La circulation de liquide ainsi obtenue depuis l'entrée dans le dispositif de traitement et jusqu'au point de distribution est illustrée par les flèches en pointillés sur la figure 4.

Cette figure 4 illustre également par des flèches en traits pleins l'irradiation du liquide par la lumière UV-C générée par la LED 22, par exemple juste avant sa distribution. Ainsi qu'on peut le voir sur cette figure 4, la surface intérieure de la chambre de traitement 6, de la tête intérieure 7 et du tube de sortie 8 réfléchit la lumière UV-C, afin de pouvoir non seulement poursuivre l'effet d'irradiation dans la chambre de traitement 6 mais ensuite aussi jusqu'au point de distribution. Pour cela, la coupelle 17 formant la chambre de traitement, la tête intérieure 7 et le tube de sortie 8 sont, ici, réalisé en un matériau réfléchissant la lumière UV, ici un fluoropolymère, et de préférence du polytétrafluoroéthylène (PTFE), offrant une réflexion diffuse très efficace et uniforme de la lumière UV et une résistance à la formation de biofilm et de calcaire sur ses surfaces.

En variante, l'on peut revêtir la surface interne de ces éléments d'un revêtement en un matériau réfléchissant les rayons UV-C.

En pratique, la LED est choisie pour émettre des rayons UV-C, c'est-à-dire des rayons ayant généralement une longueur d'onde comprise entre environ 100 nm et 400 nm, et plus particulièrement une longueur d'onde comprise entre environ 153 nm et 280 nm pour les UV-C, et de préférence une longueur d'onde comprise entre environ 240 nm et 280 nm pour assurer une meilleure capacité de décontamination (destruction des virus et bactéries présents dans le liquide).

On notera encore que (voir figure 4) l'axe de la chambre de traitement 6 et du passage d'écoulement formé par la tête intérieure 7 forme un angle α supérieur à 90°, de préférence égale à 100°, avec l'axe formé par le passage d'écoulement du tube de sortie, afin que les rayons UV arrivant sur la surface plane inclinée 15a formée par le passage 15 en forme de prisme triangulaire et faisant face au passage d'écoulement de la tête intérieure 7 cheminent sensiblement parallèlement à l'axe formé par le passage d'écoulement du tube de sortie 8, à la faveur d'une irradiation maximale du tube de sortie 8. Cette surface inclinée 15a fait elle-même, ici, un angle β de 40° avec l'axe de la chambre de traitement 6 et du passage d'écoulement formé par la tête intérieure 7.

Les sections tronconiques ménagées dans le tube de sortie 8 et dans la tête intérieure 7 permettent quant à elles de concentrer les rayons UV, au bénéfice d'une irradiation optimale du liquide.

Comment on peut encore le voir sur les figures, la LED 22 est montée en surface sur la carte électronique 4 relié pour l'alimentation électrique à un câble d'alimentation 28.

Cette carte 4 est située dans un logement étanche aux fluides 24 jouxtant la chambre de traitement 6. En pratique, l'étanchéité est obtenue par un joint d'étanchéité 25 disposé à la jonction entre l'échangeur thermique 3, formant en pratique ce logement 24, et la fenêtre en quartz 5. Du côté de cette fenêtre opposé au logement 24 , un joint d'étanchéité 26 est également prévu entre la fenêtre 5 et la coupelle 17 formant la chambre de traitement 6.

On observera encore que cet échangeur thermique 3 est ici vissé dans le corps 1, tandis que la carte électronique 4 est également vissée au moyen de vis 32 sur le fond du logement 24.

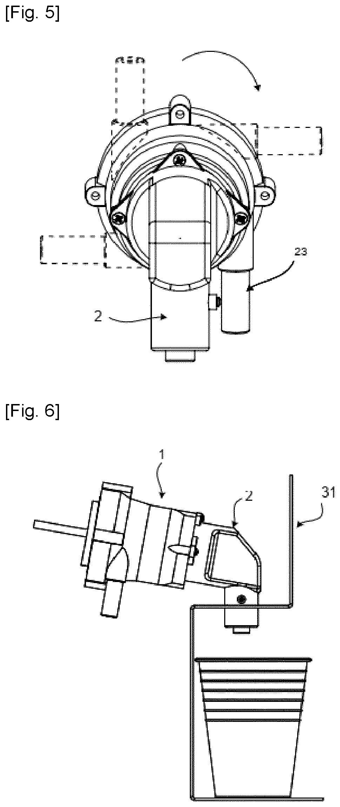

Par ailleurs, grâce à différents trous de vis 30 prévus tant sur la tête orientable 2 que sur le corps 1 de réacteur de sorte à pouvoir fixer cette tête sur le corps selon différentes positions angulaires de ce corps de réacteur, il est possible, comme illustré par la figure 5, de disposer également le tube d'entrée de liquide 23 suivant différentes positions angulaires, dictées par les possibilités de connexion des arrivées d'eau au dispositif de traitement.

Deux autres joints d'étanchéité sont par ailleurs encore prévus, l'un 29 entre la tête intérieure 7 et le corps 1 et l'autre 27 entre l'échangeur 3 et ce même corps 1, à l'extrémité d'entrée du canal de refroidissement.

On observera également sur les figures 4 et 6 que le dispositif peut aussi être monté de façon inclinée sur un appareil de distribution de boisson, ici une fontaine à eau 31, afin d'éviter ou du moins de limiter l'eau qui stagne dans le dispositif.

Il peut également être prévu un allumage cyclique de la LED pour empêcher une rétro-contamination entre le point de distribution et le reste du dispositif de traitement.

Des tests réalisés en laboratoire sur un tel dispositif de traitement ont permis de confirmer une réduction bactérienne logarithmique cible de log de 6 (réduction de 99,9999%) sur un panel de bactéries (E.Coli et Pseudomonas Aeruginosa), performance supérieure aux performances des dispositifs actuellement sur le marché, qui ont une réduction cible de log de 4.

Bien entendu, la présente invention ne se limite ni à la description précédente ni aux figures annexées, mais s'étend à toute variante à la portée de l'homme du métier. En particulier, au lieu d'une seule LED, il peut être prévu un réseau de LEDs ménagées sur la carte électronique.

## Revendications

1. Dispositif de traitement d'un liquide par rayonnement UV-C , comportant une chambre de traitement du liquide (6), au moins une LED (22) émettant de la lumière UV-C , pour irradier le liquide à traiter circulant dans la chambre de traitement (6) avec son rayonnement UV-C **caractérisé en ce qu'**il comporte une pluralité de canaux d'entrée (11) par lesquels le liquide à traiter pénètre dans la chambre de traitement (6) et un orifice de sortie (12) de la chambre de traitement (6), par lequel le liquide sort de la chambre de traitement (6) une fois traité, lesdits canaux d'entrée (11) étant disposés concentriquement autour de l'orifice de sortie de liquide (12) et suivant une configuration en tronc de cône dont la petite base fait face à l'au moins une LED (22).

2. Dispositif selon la revendication 1, **caractérisé en ce qu'**il comporte un canal de refroidissement (19) entourant un échangeur thermique (3) sur lequel est fixé une carte électronique (4) portant la au moins une LED (22), le canal de refroidissement communiquant fluidiquement avec une arrivée (21) de liquide à traiter dans le dispositif de traitement à l'une de ses extrémités et avec la pluralité de canaux d'entrée à son autre extrémité.

3. Dispositif selon la revendication 2, **caractérisé en ce que** le canal de refroidissement est formé entre un corps (1) dans lequel sont logés la chambre de traitement et l'échangeur thermique, et ces derniers.

4. Dispositif selon la revendication 3, **caractérisé en ce que** le corps présente intérieurement des nervures espaçant le corps de la chambre de traitement.

5. Dispositif selon l'une des revendications précédentes, **caractérisé en ce qu'**il comporte un passage d'écoulement de liquide traité s'étendant de l'orifice de sortie de la chambre de traitement jusqu'à un point de distribution du liquide traité.

6. Dispositif selon la revendication 5, **caractérisé en ce que** le passage d'écoulement comporte un premier tube et les canaux d'entrée sont formés par des gorges ménagées dans le bord de l'orifice de sortie et une extrémité du premier tube logée dans l'orifice.

7. Dispositif selon la revendication 5 ou 6, **caractérisé en ce que** le passage d'écoulement comporte un premier tube (7) puis un second tube (8) formant un angle avec le premier tube, et un point de distribution du liquide traité.

8. Dispositif selon l'une des revendications 6 et 7, **caractérisé en ce que** le passage d'écoulement est partiellement tronconique dans le premier tube et, le cas échéant, le second tube.

9. Dispositif selon l'une des revendications 7et 8, **caractérisé en ce que** l'axe de la partie du passage d'écoulement située dans le premier tube forme un angle supérieur à 90°, de préférence égal à 100°, avec l'axe de la partie du passage d'écoulement située dans le second tube et une surface oblique faisant face à la partie de passage d'écoulement située dans le premier tube opère la jonction entre les deux parties de passage d'écoulement.

10. Dispositif selon l'une des revendications 6 à 9, **caractérisé en ce que** le premier tube ou les premier et second tubes sont logés dans une tête de distribution montée amovible sur un corps (1) dans lequel est logé la chambre de traitement, avec possibilité de montage selon différentes positions angulaires d'un tube d'arrivée de liquide dans le dispositif, raccordé au corps.

11. Dispositif selon l'une des revendications précédentes, **caractérisé en ce que** la chambre et, le cas échéant, le passage d'écoulement, présentent une surface interne réfléchissant le rayonnement UV-C jusqu'au point de distribution.

12. Dispositif selon l'une des revendications précédentes, **caractérisé en ce que** la chambre et, le cas échéant, le passage d'écoulement, sont formés d'un matériau réfléchissant le rayonnement UV-C, de préférence un fluoropolymère, tel que du polytétrafluoroéthylène (PTFE).

13. Dispositif selon l'une des revendications précédentes, **caractérisé en ce que** la au moins une LED est située dans un logement étanche aux fluides (24) , séparé de la chambre de traitement par une fenêtre (5) transparente au rayonnement UV-C.

14. Fontaine à eau (31) comportant un dispositif de traitement selon l'une quelconques des revendications précédentes.

15. Fontaine selon la revendication 14, **caractérisée en ce que** le dispositif de traitement est monté sur la fontaine de sorte que la chambre de traitement (6) est agencée inclinée vers le bas.

16. Fontaine selon la revendication 14 ou 15, **caractérisée en ce qu'**elle comporte des moyens de commande pour un allumage cyclique de la au moins une LED (22), destiné empêcher une rétro-contamination entre le point de distribution et le reste du dispositif de traitement.
